(19) Europäisches Patentamt
European Patent Office
Office européen des brevets

(11) Publication number: **0 270 201 B1**

# (12) EUROPEAN PATENT SPECIFICATION

(45) Date of publication of patent specification: **13.03.91**

(51) Int. Cl.[5]: **C07D 239/50**

(21) Application number: **87306305.1**

(22) Date of filing: **16.07.87**

## (54) A method for the preparation of a 2,4-diamino-3-oxy-pyrimidine derivative.

(30) Priority: **07.10.86 FI 864046**

(43) Date of publication of application:
**08.06.88 Bulletin 88/23**

(45) Publication of the grant of the patent:
**13.03.91 Bulletin 91/11**

(84) Designated Contracting States:
**AT BE CH DE ES FR GB GR IT LI LU NL SE**

(56) References cited:
**GB-A- 2 032 434**

(73) Proprietor: **Orion-yhtymä Oy**
**Orionintie 1, PL 65**
**SF-02101 Espoo(FI)**

(72) Inventor: **Lamsa, Jyrki**
**Tapiontie 6B 22**
**SF-90570 Oulu(FI)**

(74) Representative: **Sexton, Jane Helen et al**
**J.A. Kemp & Co. 14 South Square Gray's Inn**
**London WC1R 5LX(GB)**

**EP 0 270 201 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

This invention relates to the preparation of intermediates useful in the preparation of 2,4-diamino-3-oxy-6-piperidylpyrimidine or minoxidil.

Minoxidil, which has the formula

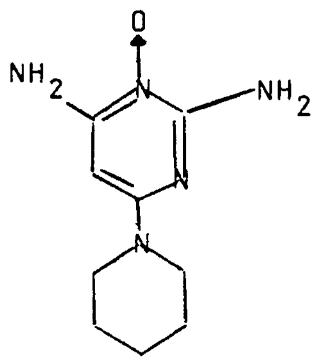

is useful as an antihypertensive agent. Methods for the preparation of minoxidil have been disclosed for example in the German patent publication DOS 1620649, US-patent 3910928, Finnish patent FI 55194 and Finnish patent application No. 793307.

The Finnish patent application 793307 describes e.g. a method for the preparation of minoxidil, which comprises reacting 6-hydroxy-2,4-diamino-pyrimidine with a sulfonyl halide of the formula R-$SO_2$-hal, wherein hal is halogen, to give a compound of the formula

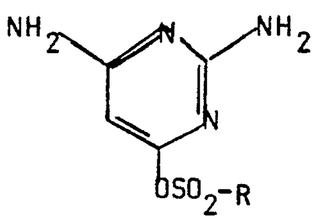

I

wherein R is a $C_{1-4}$ alkyl or phenyl which is unsubstituted or substituted by up to three $C_{1-4}$alkyl substituents.

This compound is then reacted with a peracid, for example chloroperbenozic acid, to give a compound of the formula

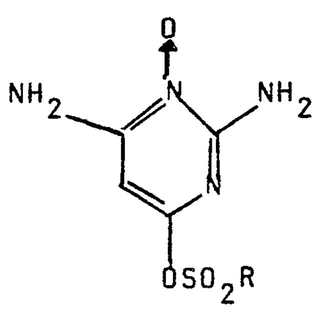

II

which is finally reacted with piperidine to give minoxidil.

This process has some disadvantages. The oxidizing agent m-chloroperbenzoic acid (or m-CPBA) is a second grade poison. When it is used it is necessary to isolate the oxidation product by a rather complicated and dangerous method. The solvent is evaporated to dryness, and the residue is dissolved in a large amount of ethyl acetate, which is extracted several times with dilute sodium hydroxide solution. Finally the ethyl acetate is dried and evaporated to a smaller volume whereon the product precipitates. Thus, when m-CPBA is used, the reaction mixture is evaporated to dryness and there is a risk that explosive peroxides may be formed during the evaporation to dryness. In addition, oxidation with m-CPBA gives relatively large

amounts of byproducts.

We have now surprisingly found that the above mentioned disadvantages can be avoided if the oxidation is effected with magnesium monoperoxyphthalate (MMPP), preferably as its hexahydrate, which has the formula

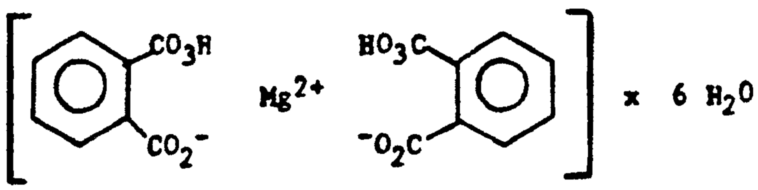

This compound is not classified as a toxic agent. It is a solid stable compound which does not explode on heating. When the oxidation is performed with this reagent, the oxidation product can be precipitated directly from the aqueous solution.

The present invention accordingly provides a method for the preparation of a compound of formula II as hereinbefore defined which comprises oxidizing a compound of formula I with magnesium monoperoxyphthalate.

In the new process the complicated and dangerous distillation to dryness is avoided. MMPP is a more reactive oxidant than m-CPBA, but does not produce byproducts to the same extent. Moreover, MMPP is cheaper than m-CPBA.

In a preferred embodiment of the new process R is p-tolyl.

The oxidation product then obtained, 6-tosyloxy-2,4-diaminopyrimidine-3-oxide, may be further reacted according to known methods to give minoxidil.

The starting material for the new process, 6-(p-tolylsulfonyloxy)-2,4-diaminopyrimidine, can be prepared, e.g., according to the method disclosed in FI application 793307.

The oxidation can be performed in various solvents. Suitable solvents are for example alcohols, preferably lower alcohols, aqueous solutions for alcohols, acetic acid, acetone, tetrahydrofuran, aqueous solution of methylene chloride and a phase transfer catalyst, DMF and DMSO. A suitable temperature is from 5 to 50° C, preferably 15-30° C. The reaction can be performed in the pH range 4 to 9.

The method of the invention is illustrated in the following Example.

## Example

a) 2,4-diamino-3-oxy-6-(p-tolylsulfonyloxy)-pyrimidine

25 g (0.089 mol) of 2,4-diamino-6-(p-tolylsulfonyloxy)-pyrimidine prepared according to the method disclosed in FI-application 793307 (example 1, step 1, method A), were suspended in a mixture containing 200 ml of acetone and 200 ml of water.

87.9 kg (0.178 mol) of magnesium monoperoxyphthalate hexahydrate (MMPP) are added to this mixture. The mixture is stirred at room temperature for 4 hours. 250 ml of water are added and the mixture is stirred at a temperature of 5-15° C for 1 h. The precipitate is filtered off and washed twice with 100 ml of water and once with 25 ml of cold methanol. 21 g (80%) of 2,4-diamino-3-oxy-6-(p-tolylsulfonyloxy)-pyrimidine are obtained. M.p. 128-129° C (decomposes).

b) 2,4-diamino-3-oxy-6-piperidylpyrimidine

20 g (0.067 mol) of 2,4-diamino-3-oxy-6-(p-tolylsulfonyloxy)-pyrimidine from the foregoing step and 140 ml of piperidine are heated at 80-90° C for 2 h. Piperidine is distilled off in vacuum. To the residue are added 50 ml of a 5% solution of sodium hyrdoxide in water and 50 ml of toluene, and the mixture is stirred at 20-25° C for half an hour. The mixture is filtered and the precipitate is washed twice with 30 ml of water and once with 30 ml of toluene. 8.4 g (60%) of 2,4-diamino-3-oxy-6-piperidylpyrimidine are obtained. M.p. 255-260° C (decomposes).

## Claims

1. A method for the preparation of a compound of the formula

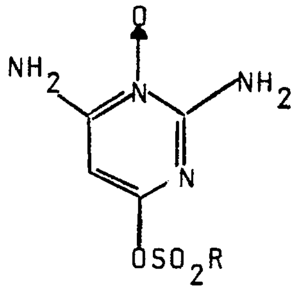

II

wherein R is a $C_{1-4}$ alkyl or a phenyl group which is unsubstituted or substituted by up to three $C_{1-4}$ alkyl substituents, which comprises oxidizing a compound of the formula

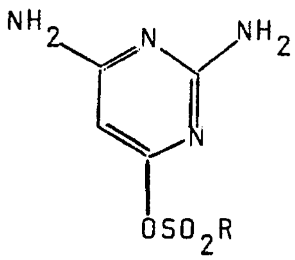

I

with magnesium monoperoxyphthalate.

2. A method according to claim 1 in which the magnesium monoperoxyphthalate is used in the form of its hexahydrate.

3. A method according to claim 1 or 2 wherein R is p-tolyl.

4. A method according to any one of claims 1 to 3 wherein the product is reacted with piperidine to give minoxidil.

## Revendications

1. Procédé de préparation d'un composé de formule :

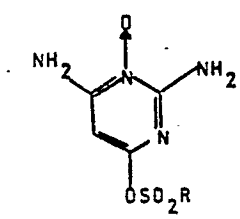

II

dans laquelle R est un groupe alkyle ayant 1 à 4 atomes de carbone ou un groupe phényle, qui est non substitué ou substitué par au maximum trois substituants alkyle ayant 1 à 4 atomes de carbone, procédé qui comprend l'oxydation d'un composé de formule :

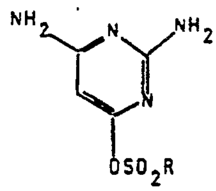

I

par du monoperoxyphtalate de magnésium.

2. Procédé selon la revendication 1, dans lequel le monoperoxyphtalate de magnésium est utilisé sous la forme de son hexahydrate.

3. Procédé selon la revendication 1 ou 2, dans lequel R est p-tolyle.

4. Procédé selon l'une quelconque des revendications 1 à 3, dans lequel le produit est mis à réagir avec la pipéridine pour donner le minoxidil.

## Ansprüche

1. Verfahren zur Herstellung einer Verbindung mit der Formel

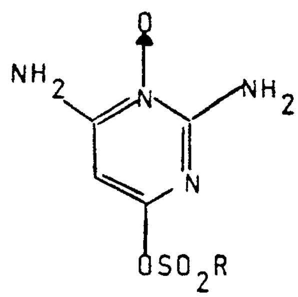

II

bei der R eine $C_{1-4}$-Alkyl- oder Phenylgruppe ist, die nicht substituiert ist oder die mit bis zu drei $C_{1-4}$-Alkylsubstituenten substituiert ist, das Oxidieren einer Verbindung mit der Formel

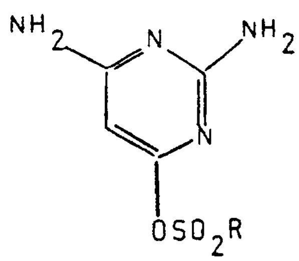

I

mit Magnesiummonoperoxiphthalat aufweist.

2. Verfahren nach Anspruch 1, bei dem das Magnesiummonoperoxiphthalat in der Form seiner Hexahydrate verwendet wird.

3. Verfahren nach Anspruch 1 oder 2, bei dem R p-Tolyl ist.

4. Verfahren nach einem der Ansprüche 1 bis 3, bei dem das Produkt mit Piperidin zum Darstellen von Minoxidil reagiert.